Europäisches Patentamt

**European Patent Office**

Office européen des brevets

(11) Veröffentlichungsnummer: **0 016 023**
**B1**

(12) **EUROPÄISCHE PATENTSCHRIFT**

(45) Veröffentlichungstag der Patentschrift:
**08.09.82**

(21) Anmeldenummer: **79900619.2**

(22) Anmeldetag: **31.05.79**

(86) Internationale Anmeldenummer:
**PCT/DE 79/00053**

(87) Internationale Veröffentlichungsnummer:
**WO 79/01155 (27.12.79 Gazette 79/27)**

(51) Int. Cl.³: **H 02 K 33/18, F 04 B 43/08,
G 05 D 16/20, A 61 M 1/03**

(54) **ELEKTROMAGNETISCHE ANTRIEBSANORDNUNG FÜR OSZILLIERENDE VERDRÄNGERPUMPEN.**

(30) Priorität: **31.05.78 DE 2823802**

(43) Veröffentlichungstag der Anmeldung:
**01.10.80 Patentblatt 80/20**

(45) Bekanntmachung des Hinweises auf die Patenterteilung:
**08.09.82 Patentblatt 82/36**

(84) Benannte Vertragsstaaten:
**FR**

(56) Entgegenhaltungen:
**DE-A-2 059 971**
**DE-A-2 614 973**
**DE-A-2 723 215**
**FR-A-1 091 752**
**FR-A-2 094 315**
**GB-A-718 199**
**GB-A-919 796**
**US-A-3 463 984**
**US-A-3 863 082**
**US-A-3 869 625**
**US-A-3 896 319**

**E & M, ELEKTROTECHNIK UND MASCHINENBAU,
Heft 1, veröffentlicht Januar 1973, Wien, O. Roubicek,
«Die Übertragungseigenschaften des Niederfrequenzsynchronlinearmotors»**

(73) Patentinhaber: **SPEIDEL & KELLER GmbH & CO. KG,
Zollerstrasse, D-7455 Jungingen (DE)**

(72) Erfinder: **BRAMM, Günter, Luisenstrasse 49,
D-8000 München 2 (DE)**
Erfinder: **NOVAK, Pavel, Görresstrasse 2,
D-8000 München 40 (DE)**

(74) Vertreter: **Reinländer & Bernhardt Patentanwälte,
Orthstrasse 12, D-8000 München 60 (DE)**

ACTORUM AG

Elektromagnetische Antriebsanordnung für oszillierende Verdrängerpumpen

Die Erfindung betrifft eine elektromagnetische Antriebsanordnung für oszillierende Verdrängerpumpen nach dem Oberbegriff des Anspruchs 1.

Solche Antriebsvorrichtungen sind bekannt (FR-OS-1 091 752; DE-OS-1 965 789). Bei diesen bekannten Antriebsvorrichtungen ist der zeitliche Verlauf des Volumenstroms und des Saugdruckes und des Ausschiebedruckes durch die Geometrie der Polschuhe und die Feldausbildung festgelegt, und damit u.a. auch die Strömungsgeschwindigkeit; die Regelung der Ausgangsgrösse bezieht sich nur auf deren Endwert (bei Amplitudenregelung) bzw. deren Durchschnittswert über mehrere Pumpzyklen.

Beim Fördern von Medien, die nur einen bestimmten Unterdruck oder Überdruck ohne Schädigung ertragen, muss bei solchen Pumpen die Antriebsgeschwindigkeit erniedrigt werden, damit auch die kurzzeitig auftretenden Unter- oder Überdrücke die zulässigen Grenzwerte nicht überschreiten. Dadruch wird der Mengenstrom erheblich erniedrigt und die Pumpe nur schlecht ausgenützt. Zur Verringerung dieser Schwierigkeiten können zwar Druck- und Volumenspeicher in Form von Windkesseln vor- oder nachgeschaltet werden. Dadurch wird die gesamte Pumpanlage aufwendiger und teurer. Ausserdem kann dadurch nicht ausgeschlossen werden, dass dennoch die zulässigen Unter- oder Überdrücke überschritten werden. Ausserdem erhöht sich dadurch das in der Pumpanlage befindliche Volumen des Fördermediums.

Bei Fördermedien, bei denen bestimmte maximale Scherspannungen nicht überschritten werden dürfen, müssen die Beschleunigungskräfte der bewegten Pumpenteile so niedrig gehalten werden, dass der Grenzwert der Scherspannung an jeder Stelle des hydraulischen Systems auch kurzzeitig nicht überschritten wird. Auch dadurch wird der Ausnutzungsgrad der Pumpe stark erniedrigt.

Aufgabe der Erfindung ist es deshalb, die eingangs genannte Antriebsvorrichtung derart auszubilden, dass zwar einerseits die zulässigen Grenzwerte für Unterdruck, Überdruck, Beschleunigung etc. sicher eingehalten, d.h. auch nicht kurzzeitig überschritten, werden, andererseits aber innerhalb der Genzwerte die grösstmögliche Förderleistung erreicht wird.

Erfindungsgemäss wird diese Aufgabe durch die im Kennzeichenteil des Anspruchs 1 aufgeführten Merkmale gelöst.

Schwingankermotoren, bei denen die Wicklung eine auf dem Läufer aufgebrachte Tauchspulenwicklung ist, die sich in einem vom Stator gebildeten Magnetfeld bewegen kann, sind bekannt (GB-PS-718 199, 14 42 500; US-PS-38 63 082, 38 96 319). Die geringe Masse eines Läufers mit Tauchspulenwicklung ist jedoch nicht zur Ausregelung der Ausgangsgrösse während der Bewegung der Tauchspule innerhalb eines Hubes ausgenutzt worden.

Es ist zwar bekannt, bei Positionierern für Plattenspeicher für die Datenverarbeitung, die elektrodynamische Linearmotoren aufweisen, eine Steuerschaltung vorzusehen, die in Abhängigkeit von der Differenz zwischen dem Ist-Wert des Positionierweges und dem Soll-Wert der gewünschten Position sowie in Abhängigkeit von der Positioniergeschwindigkeit den Positionierhub steuert, bei solchen Positionierern für Plattenspeicher für die elektronische Datenverarbeitung treten jedoch ganz andersgeartete Probleme auf, insbesondere kommt es darauf an, dass die gewünschte Soll-Position präzise erreicht wird, so dass diese bekannte Anordnung für Antriebsvorrichtungen für oszillierende Verdrängerpumpen keine Anregung liefern konnte (Feinwerktechnik und Mikronik 77 (1973), Heft 4, Seite 151–157).

Spezielle Ausgestaltungen der Erfindung ergeben sich aus den abhängigen Patentansprüchen, deren Merkmale zum Teil an sich bekannt sind. So ermöglicht eine Antriebsvorrichtung, bei der als Signalgeber ein Weggeber eingesetzt ist, den Einsatz der angetriebenen Verdrängerpumpe als Dosierpumpe. Liefert der Signalgeber ein von der Geschwindigkeit abhängiges Signal (Weggeber mit nachgeschaltetem Differenzierglied oder Geschwindigkeitsgeber), so ist die angetriebene Verdrängerpumpe besonders zum Fördern von Medien geeignet, die gegen hohe Scherspannungen empfindlich sind, wie etwa Blut. Wird als Signalgeber ein Druckwandler verwendet, kann das Überschreiten eines bestimmten Überdruckes und/oder das Unterschreiten eines bestimmten Unterdruckes vermieden werden, so dass die angetriebene Verdrängerpumpe besonders für druckempfindliche Medien oder für Medien geeignet ist, die zum Ausgasen neigen. Ist der (Geschwindigkeits-) Sollwertgeber an zwei Endstellungswertgeber für die beiden Umkehrschaltungen des Läufers angeschlossen, kann bei veränderlicher Fördergeschwindigkeit die Pumpfrequenz konstanz gehalten werden, was vor allem für den Einsatz der angetriebenen Pumpe als Blutpumpe vorteilhaft ist.

Wird der Läufer am Polkern eines entsprechend gestalteten Ständers geführt, wird eine denkbar einfache Längsführung des Läufers erreicht, die ohnehin vorhandene Teile ausnutzt, und wenn diese Führung mit Wälzlagern ausgestattet wird, ergibt sich darüber hinaus noch eine reibungsarme und damit verlustleistungsarme Führung.

Die Führung des Läufers eines Tauchspulenmotors ist an sich bekannt (US-PS-3 863 082, 3 869 625).

Beim Aufbau der Vorrichtung nach Anspruch 16 ergibt sich eine sehr kompakte Einheit aus Antriebsvorrichtung und Verdrängerpumpe, wobei bei Weiterbildung nach Anspruch 17 eine besonders steife Koppelvorrichtung mit einer hohen Festigkeit und einer günstigen Werkstoffausnutzung der Teile erreicht wird. Mit einer Ausgestaltung der Verdrängerpumpe nach Anspruch 18 wird sowohl der Zusammenbau der Pumpe als auch ihre Wartung sehr vereinfacht. Auch lassen sich dadurch die gleichen Teile untereinander leichter austauschen. Eine Ausgestaltung nach Anspruch 19 ergibt eine vielseitig einsetzbare Verdrängerpumpe, mit der auch besonders schwierige, etwa stark klebende oder gegen übliche Dichtungsmedien aggressive oder gegen diese empfindliche Medien gefördert werden können.

Die Weiterbildung nach Anspruch 20 ermöglicht es, den Pumpenteil der Verdrängerpumpe aus einem vielseitig einsetzbaren Werkstoff verhältnismässig billig herzustellen, vor allem lassen sich bei Innendurchfluss auch Toträume, in denen das gepumpte Medium stagnieren kann, weitgehend ausschalten. Bei schwierig zu entfernenden Medien oder bei hohen Anforderungen an die Sauberkeit des Pumpenteils, beispielsweise an die Keimfreiheit bei der Verwendung als Blutpumpe, kann der Pumpenteil ohne grossen Kostenaufwand als sog. Wegwerfteil behandelt werden. Bei einer Ausgestaltung der Antriebsvorrichtung und der Verdrängerpumpe nach einem der Ansprüche 21 oder 22 lassen sich die beiden Pumpenteile sehr leicht zusammenfügen und auch wieder voneinander trennen. Dadurch wird ebenfalls die Behandlung des Pumpenteil als Wegwerfteil erleichtert.

Im folgenden wird die Erfindung anhand eines in den Zeichnungen dargestellten Ausführungsbeispieles einer Antriebseinheit mit einer oszillierenden Verdrängerpumpe und anhand mehrerer Steuerschaltungen dafür näher erläutert. Es zeigen:

Fig. 1 einen teilweise schematisch dargestellten Vertikalschnitt durch eine Antriebseinrichtung und eine Balgpumpe nach der Linie I–I in Fig. 2;

Fig. 2 eine Draufsicht auf die Einrichtung nach Fig. 1 bei abgenommener oberer Jochscheibe;

Fig. 3 bis 6 Blockschaltbilder verschiedener Steuerschaltungen für die Einrichtung nach Fig. 1 und 2;

Fig. 7 einen Schaltplan des Blockschaltbildes nach Fig. 6.

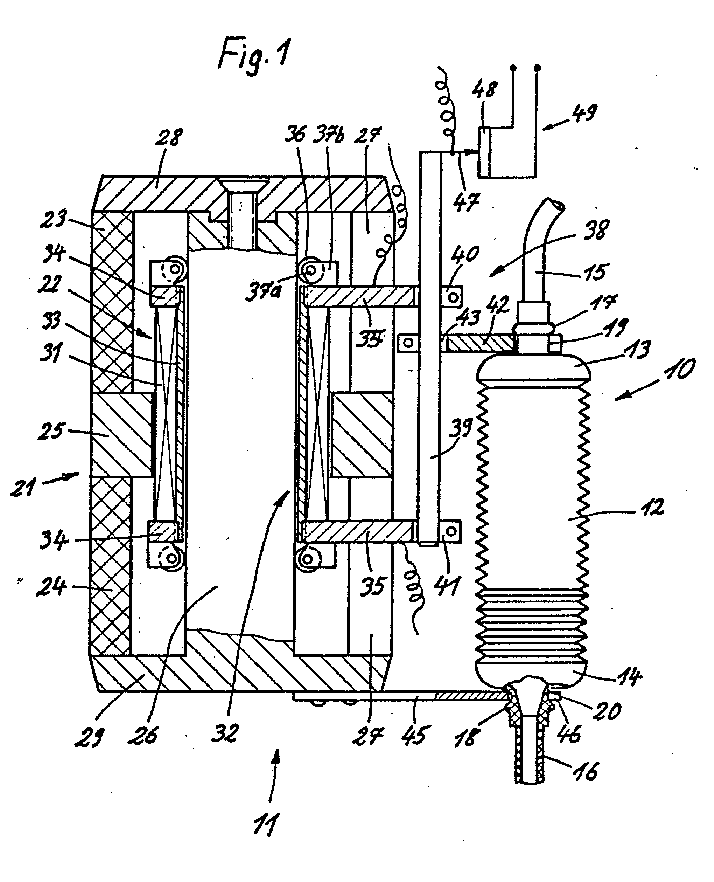

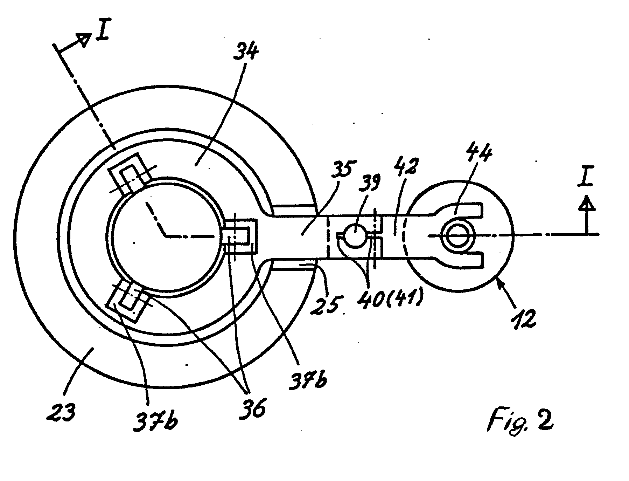

Die aus Fig. 1 und 2 ersichtliche oszillierende Verdrängerpumpe weist als Hauptbaugruppen eine Balgpumpe 10 und eine elektromagnetische Antriebseinrichtung 11 auf.

Die Balgpumpe 10 ist als Pumpe mit Innendurchfluss ausgebildet. Sie weist einen Faltenbalg 12 von zylindrischer Form auf, der an den beiden Stirnseiten in je einen glatten gewölbten kreisringförmigen Deckelteil 13 bzw. 14 übergeht. In der Mitte der Deckelteile 13 und 14 schliesst sich nahtlos je ein Schlauchteil 15 bzw. 16 an. An der Übergangsstelle zwischen dem Deckelteil 13 und 14 und dem zugehörigen Schlauchteil 15 bzw. 16 ist je ein Ringwulst 17 bzw. 18 vorhanden, der in radialer Richtung über die axial beiderseits benachbarten Teile übersteht, sodass zwischen dem Ringwulst 17 und 18 und dem zugehörigen Deckelteil 13 bzw. 14 eine Ringrille 19 bzw. 20 gebildet wird.

Der Faltenbalg 12, die beiden Deckelteile 13 und 14, die daran anschliessenden Schlauchteile 15 und 16 und die Ringwülste 17 und 18 sind als einstückiges Formteil aus einem gummielastischen Werkstoff, beispielsweise aus Polyäthylen oder aus Polyvinylchlorid, hergestellt.

Die für eine fortwährende Förderung eines Mediums durch die Pumpe hindurch erforderlichen Ventile sind in Fig. 1 nicht dargestellt. Sie können entweder als passive Ventile, etwa als federbelastete Rückschlagventile, ausgebildet sein, die mit Schlauchstutzen an den Schlauchteilen 15 und 16 angeschlossen werden und durch das strömende Medium selbst betätigt werden. Diese Ventile können aber auch als aktive Ventile, etwa als elektromagnetisch betätigte Schlauchventile ausgebildet sein, die ausserhalb der Schlauchteile 15 und 16 angeordnet sind und als sogenannte Quetschhähne oder Quetschventile von aussen her auf die Schlauchteile einwirken.

Die elektromagnetische Antriebseinrichtung 11 weist einen Ständer 21 und einen Läufer 22 auf. Der Ständer weist für die Erzeugung des magnetischen Ständerfeldes zwei Dauermagnetsysteme 23 und 24 auf, die beide hohlzylinderförmig ausgebildet sind, wie aus Fig. 2 ersichtlich ist. Die beiden Dauermagnetsysteme 23 und 24 sind aus einer Aluminium-Nickel-Kobalt-Legierung hergestellt. An der in Fig. 1 und 2 rechts gelegenen Seite ist die Ringform der Hohlzylinder der Dauermagnetsysteme 23 und 24 durch je eine radial und axial durchgehende Aussparung 27 unterbrochen, die von zueinander parallel ausgerichteten ebenen Wandflächen begrenzt wird. Die Magnetfelder der beiden Dauermagnetsysteme 23 und 24 sind einander entgegengesetzt ausgerichtet.

Zwischen den beiden Dauermagnetsystemen 23 und 24 befindet sich ein kreisförmiger Polschuh 25, an dessen axialen Stirnseiten die Dauermagnetsysteme 23 und 24 anliegen. Mittig innerhalb des Polschuhs 25 und der Dauermagnetsysteme 23 und 24 ist ein zylindrischer Polkern 26 mit kreisförmigem Querschnitt angeordnet. Zwischen dem ringförmigen Polschuh 25 und dem zylindrischen Polkern 26 ist ein ringförmiger Polspalt von gleichbleibender Spaltweite vorhanden. Der Polkern 26 hat die gleiche axiale Erstreckung, wie die Summe der axialen Erstreckungen des Polschuhs 25 und der beiden Dauermagnetsysteme 23 und 24. An den beiden Enden schliessen an den Polkern je eine ebene kreisförmige Jochscheibe 28 bzw. 29 an. Die in Fig. 1 oben gelegene Jochscheibe 28 ist als Einzelteil ausgebildet und mit dem Polkern 26 verschraubt. Die unten gelegene Jochscheibe 29 ist zusammen mit dem Polkern 26 einstückig ausgebildet. Denkbar wäre auch, dass beide Polscheiben als Einzelteile hergestellt und mit dem Polkern verschraubt werden oder dass der Polkern etwa in der Längsmitte geteilt ist und an jedem Ende die Polscheibe einstückig angeformt ist.

Der ringförmige Polschuh 25, der Polkern 26 und die beiden Jochscheiben 28 und 29 sind aus einem weichmagnetischen ferromagnetischen Werkstoff, zum Beispiel aus einer Eisen-Kobalt-Legierung, hergestellt. Die axiale Erstreckung des Polschuhs 25 und des Polkerns 26 sind aufeinander und auf die axiale Erstreckung der beiden Dauermagnetsysteme 23 und 24 so abgestimmt, dass im zusammengefügten Zustand kein Luftspalt zwischen diesen Teilen vorhanden ist.

Der Läufer 22 ist als nicht-ferromagnetischer Körper ausgebildet. Er wird durch eine Wicklung 31 und durch einen Wicklungsträger 32 gebildet. Der Wicklungsträger 32 ist aus einer Hülse 33 und aus zwei Endscheiben 34 gebildet. Die Hülse 33 hat die Form eines dünnen Hohlzylinders. Die beiden Endscheiben 34 sind kreisförmig ausgebildet und haben einen nach aussen abstehenden radialen Fortsatz, der je einen Kragarm 35 des Läufers 22 bildet.

Die axiale Erstreckung des Polschuhs 25, des Ständers 21 und die axiale Erstreckung der Wicklung 31 des Läufers 22 sowie der grösste im Betrieb auftretende Hub des Läufers 22 und damit der Balgpumpe 10 sind so aufeinander abgestimmt, dass die axiale Erstreckung der Wicklung 31 zumindest annähernd gleich der Summe aus der axialen Erstreckung des Polschuhs 25 und des grössten Betriebshubes des Läufers 22 ist. Der grösste Betriebshub wird in der Regel durch die Anforderungen an die Balgpumpe 10 vorgegeben. Die axiale Erstreckung des Polschuhs 25 ergibt sich im wesentlichen aus den magnetischen Eigenschaften der an der Erzeugung des Ständerfeldes beteiligten Teile. Danach richtet sich dann die axiale Erstreckung der Wicklung 31. Infolge dieser Bemessung tauchen bei einem vollen Hub des Läufers 22 sämtliche Windungen der Wicklung 31 wenigstens einmal in das radiale Ständerfeld im Polspalt zwischen dem Polschuh 25 und dem Polkern 26 ein und keine der Windungen läuft völlig leer mit ohne jemals an der Krafterzeugung der Antriebseinrichtung 11 mitzuwirken und lediglich Energieverluste hervorzurufen.

Der Läufer 22 ist am Polkern 26 geführt, und zwar, mittels zweier Gruppen von je drei Wälzlagern 36, die am Umfang des Läufers 22 gleichmässig verteilt angeordnet sind, wie aus Fig. 2 ersichtlich ist. Jede Wälzlagergruppe ist an je einer der Stirnseiten des Wicklungsträgers 32 angeordnet. Die Innenringe der Wälzlager 36 sitzen auf Lagerstiften 37a, die ihrerseits in kleinen Lagerböcken 37b eingesteckt sind. Diese Lagerböcke 37b sind auf den aussen gelegenen Stirnseiten der beiden Endscheiben 34 mittels nicht dargestellter Schrauben festgeschraubt. Die Aussenringe der Wälzlager 36 laufen unmittelbar auf der Umfangsfläche des Polkerns 26. Die Kragarme 35 sind Teil einer Koppelvorrichtung 38 zwischen der Balgpumpe 10 und ihrer Antriebseinrichtung 11. Als weiterer Teil der Koppelvorrichtung 38 ist ein Bügel 39 vorhanden, der als zylindrischer Stab mit Kreisquerschnitt ausgebildet ist. Dieser stabförmige Bügel 39 ist in je ein kreisrundes Durchgangsloch in den Kragarmen 35 eingesteckt und darin mittels je eines radial verlaufenden Klemmschlitzes 40 bzw. 41 und eine in Umfangsrichtung eingeschraubte, nicht dargestellte Klemmschraube festgeklemmt. Als weiterer Teil der Koppelvorrichtung 38 ist eine Lasche 42 am Bügel 39 festgeklemmt und zwar ebenfalls mittels eines Klemmschlitzes 43 und einer im einzelnen nicht dargestellten Klemmschraube. Dadurch kann die Lasche 42 am Bügel 39 leicht auf den durch die Abmessungen des Pumpenbalges 12 und auf dessen Dehnungszustand in der Ausgangslage des Läufers 22 der Antriebseinrichtung 11 eingestellt werden. Die Lasche 42 weist auf der vom Bügel 39 abgekehrten Seite ein gabelförmiges Ende 44 auf. Dieses gabelförmige Ende ist in seinen Abmessungen auf die Abmessungen der Ringrille 19 an dem in Fig. 1 oben gelegenen Ende des Faltenbalges 12 abgestimmt. Als weiterer Teil der Koppelvorrichtung 38 ist eine zweite Lasche 45 vorhanden, die an der unten gelegenen Jochscheibe 29 angeschraubt ist. Auch die Lasche 45 hat auf der von der Jochscheibe 29 abgekehrten Seite ein gabelförmiges Ende 46, das in gleicher Weise, wie das gabelförmige Ende 44 der Lasche 42 auf die Abmessungen der Ringrille 20 am unteren Ende des Faltenbalges 12 abgestimmt ist.

Wie in Fig. 2 angedeutet und in Fig. 1 deutlich erkennbar ist, ist der Faltenbalg 12 der Balgpumpe 10 mit der Antriebseinrichtung 11 so zusammengefügt, dass das gabelförmige Ende 44 der Lasche 42 in die Ringrille 19 am oberen Ende des Faltenbalges 12 und das gabelförmige Ende 46 der Lasche 45 in die Ringrille 20 am unteren Ende des Faltenbalges 12 eingreifen. Durch die axiale Anlage des gabelförmigen Endes 44 der Lasche 42 an den an die Ringrille 19 beiderseits anschliessenden Teilen des Faltenbalges 12, dem Deckelteil 13 und dem Ringwulst 17, und durch die axiale Analge des gabelförmigen Endes 46 der Lasche 45 an den an die Ringrille 20 beiderseits anschliessenden Teilen des Faltenbalges 12, dem Deckelteil 14 und dem Ringwulst 18, überträgt die Koppelvorrichtung 38 die Hubbewegungen der relativ zueinander bewegbaren Teile der Antriebseinrichtung 11 auf die Balgpumpe 10.

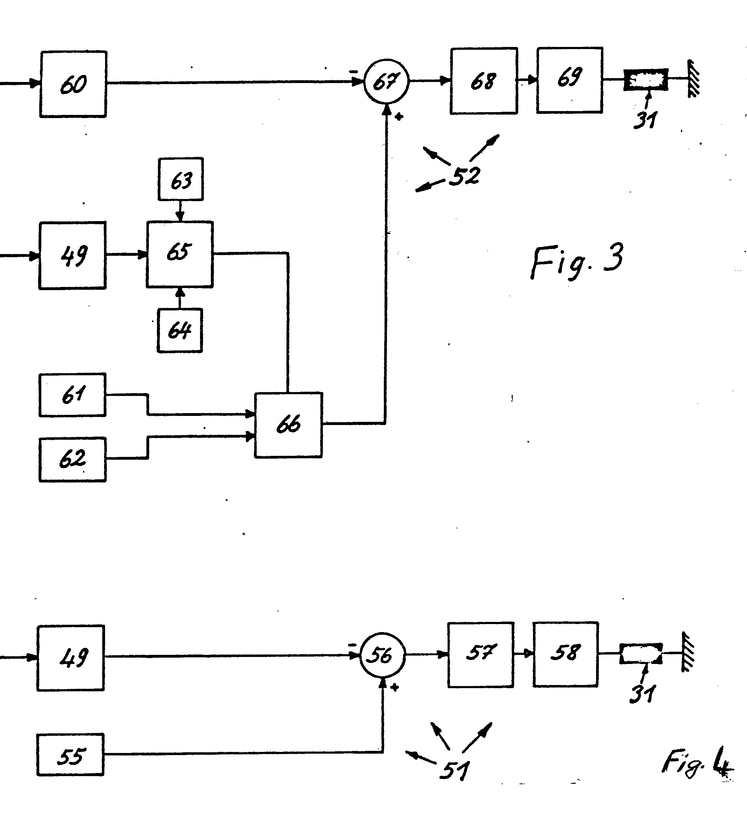

Wie aus Fig. 1 ersichtlich ist, ragt der stangenförmige Bügel 39 über den Kragarm 36 nach oben hinaus. An diesem überstehenden Ende ist eine Schleiffeder 47 angebracht, die an einem Schleifwiderstand 48 anliegt und im Zuge der Hubbewegungen des Bügels 39 auf dem Schleifwiderstand hin und her gleitet. Der Schleifwiderstand 48 ist in nicht dargestellter Weise in bezug auf den Ständer 21 der Antriebseinrichtung 11 ortsfest angeordnet. Die Schleiffeder 47 und der Schleifwiderstand 48 bilden einen Signalgeber, und zwar einen Weggeber 49, als Teil einer der Steuerschaltungen 51, 52, 53 oder 54, die nachfolgend anhand der Fig. 3 bis 6 erläutert werden.

Die aus Fig. 3 ersichtliche Steuerschaltung 51 dient der Weg- oder Hubregelung der Antriebseinrichtung 11 und damit der Balgpumpe 10.

Die Steuerschaltung 51 weist neben dem Weggeber 49 einen Sollwertgeber 55 für die Sollwegstrecke des Läufers 22, ein Vergleichsglied 56, ein Reglerglied 57 mit einem PI-Regler und eine Leistungsendstufe 58 auf. Der Sollwertgeber 55 erzeugt ein Weg-Zeit-Signal, dem der Läufer 22 mit seiner Hubbewegung folgen soll. Der Ausgang des Weggebers 49 und des Sollwertgebers 55 sind an die beiden Eingänge des Vergleichsgliedes angeschlossen und zwar mit einander entgegengesetzten Vorzeichen. Das Vergleichsglied bildet aus den beiden Eingangssignalen ein Summensignal. Der Ausgang des Vergleichsgliedes 56 ist an den Eingang des Reglergliedes angeschlossen und dessen Ausgang an den Eingang der Leistungsendstufe 58. Die Leistungsendstufe liefert das Stellsignal an die an ihren Ausgang angeschlossene Wicklung 31 des Läufers 22.

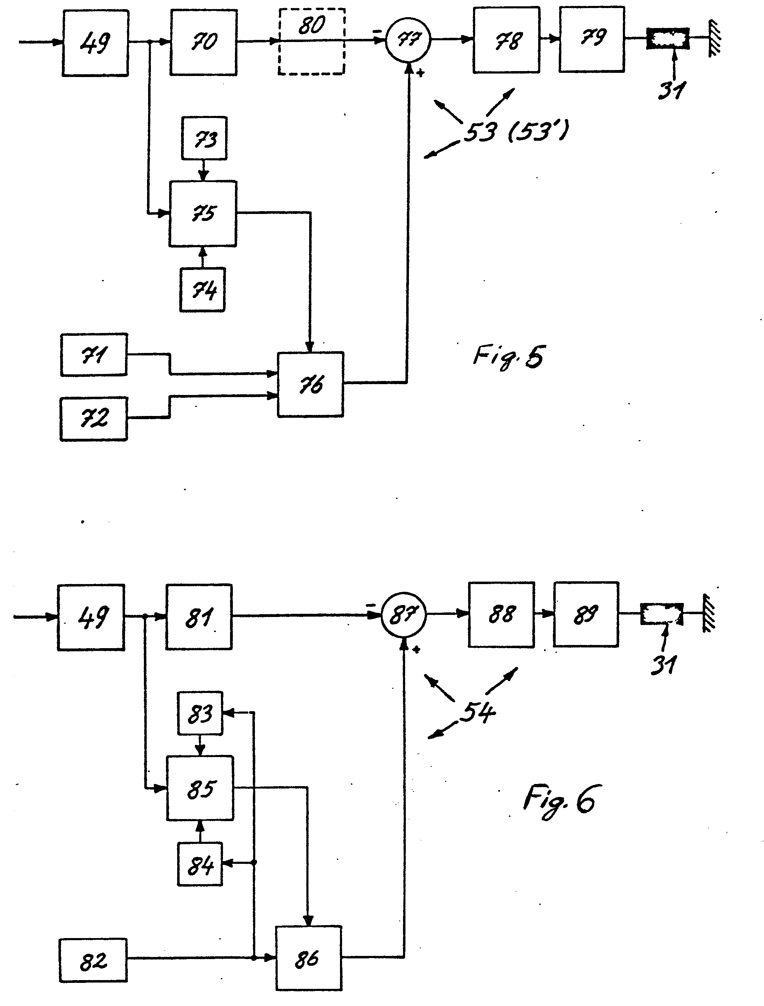

Die aus Fig. 4 ersichtliche Steuerschaltung 52 dient der Druckregelung in der Balgpumpe 10 und/oder im angeschlossenen hydraulischen System.

Die Steuerschaltung 52 weist neben dem Weggeber 49 einen Druckwandler 60, je einen Sollwertgeber 61 bzw. 62 für den Solldruck bei je einer der beiden Bewegungsrichtungen des Läufers 22, je einen Endstellungswertgeber 63 bzw. 64 für die beiden Umkehrstellungen des Läufers 22, einen Komparator 65, einen Richtungsumkehrschalter 66, ein Vergleichsglied 67, und wie zuvor ein Reglerglied 68 mit einem PI-Regler und eine Leistungsendstufe 69 auf. Der Ausgang des Weggebers 49 ist an den Signalleitungseingang des Komparators 65 angeschlossen. Ausserdem ist der Ausgang eines jeden der beiden Endstellungswertgebers 63 und 64 an die Steuerleitungseingänge des Komparators 65 angeschlossen. Dessen Ausgang ist an den Steuerleitungseingang des Richtungsumkehrschalters 66 angeschlossen. Die Ausgänge der beiden Sollwertgeber 61 und 62 sind an die Signalleitungseingänge des Richtungsumkehrschalters 66 angeschlossen. Dessen Ausgang und der Ausgang des Druckwandlers 60 sind, mit entgegengesetzten Vorzeichen, an die beiden Eingänge des Vergleichsgliedes 67 angeschlossen. Dessen Ausgang ist an den Eingang des Reglergliedes 68 und dessen Ausgang wiederum an den Eingang der Leistungsendstufe 69 angeschlossen. Diese gibt gegebenenfalls ein Stellsignal an die Wicklung 31 des Läufers 22 ab.

Die aus Fig. 5 ersichtliche Steuerschaltung 53 dient der Geschwindigkeitsregelung und mit einer Ergänzung auch der Beschleunigungsregelung der Antriebseinrichtung 11.

Die Steuerschaltung 53 weist neben dem Weggeber 49 ein Differenzierglied 70, je einen Sollwertgeber 71 bzw. 72 für die Sollgeschwindigkeit in je einer der Bewegungsrichtungen des Läufers 22, je einen Endstellungswertgeber 73 bzw. 74 für die beiden Umkehrstellungen des Läufers 22, einen Komparator 75, einen Richtungsumkehrschalter 76, ein Vergleichsglied 77, ein Reglerglied 78 mit einem PI-Regler und eine Leistungsendstufe 79 auf. Der Ausgang des Weggebers 49 ist an den Eingang des Differenziergliedes 70 und an den Signalleitungseingang des Komparators 75 angeschlossen. Der Ausgang eines jeden der beiden Endstellungswertgeber 73 und 74 ist an die Steuerleitungseingänge des Komparators 75 angeschlossen. Dessen Ausgang ist an den Steuerleitungseingang des Richtungsumkehrschalters 76 angeschlossen. Die Ausgänge der Sollwertgeber 71 und 72 sind an die Signalleitungseingänge des Richtungsumkehrschalters 76 angeschlossen. Dessen Signalleitungsausgang sowie der Ausgang des Differenziergliedes 70 sind, mit entgegengesetztem Vorzeichen, an die Eingänge des Vergleichsgliedes 77 angeschlossen. Dessen Ausgang ist an den Eingang des Reglergliedes 78 und dessen Ausgang an den Eingang der Leistungsendstufe 79 angeschlossen. Diese speist die Wicklung 31 des Läufers 22 mit einem für die Geschwindigkeitsregelung erforderlichen Stellsignal.

Wenn in der zuvor erläuterten Steuerschaltung 53 zwischen dem Differenzierglied 70 und dem Vergleichsglied 77 noch ein zweites Differenzierglied 80 eingeschaltet wird, entspricht dessen Ausgangssignal der zweiten Ableitung des Wegsignales nach der Zeit, also dem Istsignal der Beschleunigung des Läufers 22 der Antriebseinrichtung 11. Wenn die beiden Sollwertgeber 71 und 72 auf die Abgabe je eines Sollwertsignals für die Beschleunigung in je einer der beiden Bewegungsrichtungen des Läufers 22 eingestellt sind, arbeitet die derart abgewandelte Steuerschaltung 53' als Regelkreis für die Beschleunigung der Antriebseinrichtung 11.

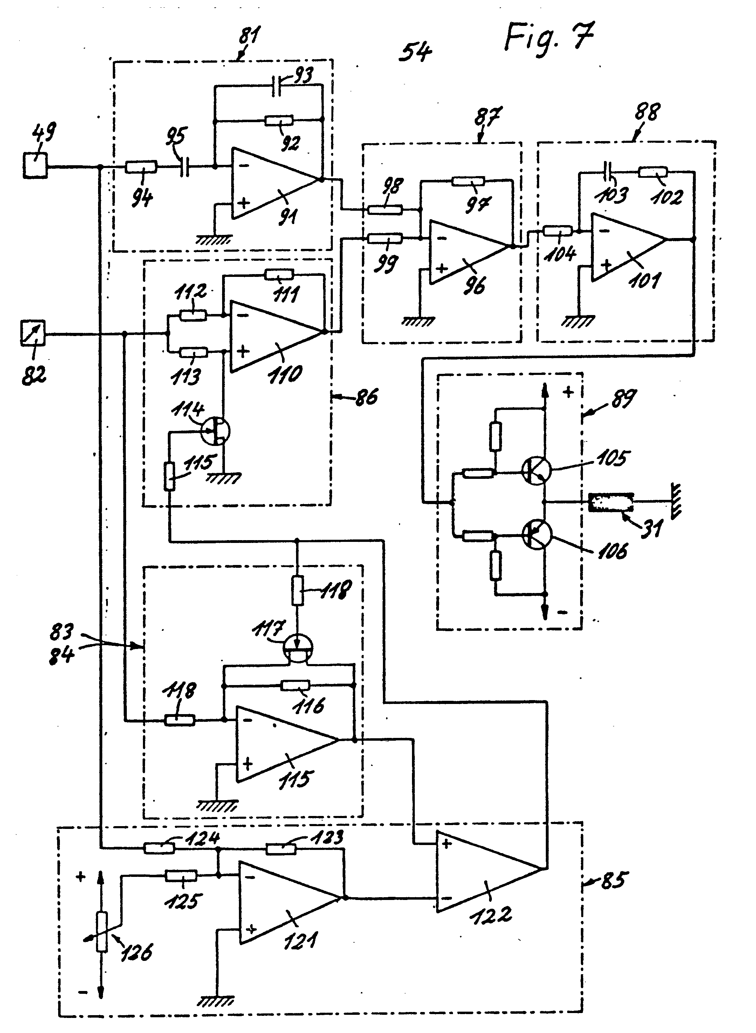

Die aus Fig. 6 ersichtliche Steuerschaltung 54 dient der Regelung und damit der Einhaltung einer konstanten Hubfrequenz der Balgpumpe 10 und ihrer Antriebseinrichtung 11 bei sich ändernder Hubgeschwindigkeit.

Die Steuerschaltung 54 weist neben dem Weggeber 49 ein Differenzierglied 81, einen Sollwertgeber 82 für die Sollgeschwindigkeit in beiden Bewegungsrichtungen des Läufers 22, je einen Endstellungswertgeber 83 bzw. 84 für die beiden Umkehrstellungen des Läufers 22, einen Komparator 85, einen Richtungsumkehrschalter 86, ein Vergleichsglied 87, ein Reglerglied 88 mit einem PI-Regler und eine Leistungsendstufe 89 auf. Der Ausgang des Weggebers 49 ist an den Eingang des Differenziergliedes 81 und zugleich an den Signalleitungseingang des Komparators 85 angeschlossen. Der Ausgang des Sollwertgebers 82 ist einmal an den Signalleitungseingang des Richtungsumkehrschalters 86 angeschlossen und einmal an je einen Steuerleitungseingang jedes der beiden Endstellungswertgeber 83 und 84 angeschlossen. Deren Ausgang ist an je einen der Steuerleitungseingänge des Komparators 85 angeschlossen. Dessen Ausgang ist an den Steuerleitungseingang des Richtungsumkehrschalters 86 angeschlossen. Dessen Signalleitungsausgang und der Ausgang des Differenziergliedes 81 sind, wiederum mit entgegengesetztem Vorzeichen, an die Eingänge des Vergleichsgliedes 87 angeschlossen. Dessen Ausgang liegt am Eingang des Reglergliedes 88 und dessen Ausgang ist an der Leistungsendstufe 89 angeschlossen, die die Wicklung 31 des Läufers 20 speist. Durch die Abgabe des Ausgangssignals des Geschwindigkeits-Sollwertgebers 82 auch an die Steuerleitungseingänge der Endstellungswertgeber 83 und 84 werden bei einer aus irgend einem Grunde auftretenden Änderung der Hubgeschwindigkeit der Antriebseinrichtung die Endstellungsgrenzwerte des Hubes entsprechend verlegt, so dass bei höherer Hubgeschwindigkeit ein längerer Hub zurückgelegt wird und umgekehrt, so dass, innerhalb bestimmter Grenzen für den Hub und damit für die Geschwindigkeit, die Hubfrequenz des gesamten oszillierenden Systems gleichbleibt.

Zur Verdeutlichung der Schaltpläne einer der Steuerschaltungen 51 bis 54 wird anhand der Fig. 7 der Schaltplan für die zuletzt abgehandelte Steuerschaltung 54 näher dargelegt.

Das Differenzierglied 81 weist einen Operationsverstärker 91 auf, der in einem Gegenkopplungskreis einen Widerstand 92 und einen Kondensator 93 hat, die zueinander parallel geschaltet sind und dessen negativem Eingang ein Widerstand 94 und ein Kondensator 95 in Reihe vorgeschaltet sind. Durch den Widerstand 92 im Gegenkopplungskreis und durch den Kondensator 95 in der Eingangsleitung wirkt der Operationsverstärker 91 als Differenzierglied. Der Widerstand 94 und der Kondensator 93 wirken mit dem Operationsverstärker zusammen zugleich als Tiefpass.

Das Vergleichsglied 87 weist einen Operationsverstärker 96 auf, der in einem Gegenkopplungskreis einen Widerstand 97 hat. Am negativen Eingang des Operationsverstärkers 96 sind ausserdem zwei Widerstände 98 und 99 parallel zueinander angeschlossen. An den Widerstand 98 ist der Signalleitungsausgang des Differenziergliedes 81 und an den Widerstand 99 ist der Signalleitungsausgang des Richtungsumkehrschalters 86 angeschlossen. Dadurch gibt das Vergleichsglied ein Summensignal aus den beiden Eingangssignalen ab.

Das Reglerglied 88 weist einen Operationsverstärker 101 auf, der in einem Gegenkopplungskreis einen Widerstand 102 und einen Kondensator 103 hat, die in Reihe geschaltet sind. Am negativen Eingang des Operationsverstärkers 101 ist ausserdem ein Widerstand 104 angeschlossen. An diesen Widerstand 104 ist der Ausgang des Vergleichsgliedes 87 angeschlossen. Durch diese Schaltung weist der Operationsverstärker 101 eine proportional und zugleich integrierend wirkende Übertragsfunktion auf.

Die Leistungsendstufe 89 weist zwei komplementäre Transistoren 105 und 106 auf, die als Gegentaktstromverstärker im A-B-Betrieb arbeiten.

Der Richtungsumkehrschalter 86 weist einen Operationsverstärker 110 auf, der im Gegenkopplungskreis einen Widerstand 111 hat. Am negativen Eingang ist ausserdem ein Widerstand 112 angeschlossen. Am positiven Eingang ist ein Widerstand 113 angeschlossen. Am positiven Eingang des Operationsverstärkers 110 ist ausserdem der Drain-Anschluss eines Feldeffekttransistors (FET) 114 angeschlossen. Der Source-Anschluss des FET 114 ist an die Erde angeschlossen. Die Gate-Elektrode des FET 114 bildet zusammen mit einem Vorwiderstand 115 den Steuerleitungseingang des Richtungsumkehrschalters 86. Daran ist der Ausgang des Komparators 85 angeschlossen. Je nach der Ausgangsspannung des Komparators 85 erscheint der Sollwert der Geschwindigkeit am Ausgang des Richtungsumkehrschalters 86 mit positivem oder negativem Vorzeichen.

Der Endstellungswertgeber 83, 84 weist einen Operationsverstärker 115 auf, der im Gegenkopplungskreis einen Widerstand 116 und parallel dazu einen FET 117 hat. An der Gate-Elektrode des FET 117 ist über einen Vorwiderstand 118 die Ausgangsspannung des Komparators 85 angelegt. Am negativen Eingang des Operationsverstärkers 115 ist ausserdem ein Widerstand 119 angeschlossen, an den seinerseits der Ausgang des Sollwertgebers 82 für die Geschwindigkeit angeschlossen ist. Am Ausgang des Endstellungswertgebers 83, 84 erscheint je nach der Ausgangsspannung des Komparators 85 der eine oder der andere Endstellungswert des Weggebers 49. Dabei ist wegen des Anschlusses des Sollwertgebers 82 für die Geschwindigkeit am Widerstand 119, dem Steuerleitungseingang des Endstellungswertgebers 83, 84, der Endstellungswert proportional der Sollgeschwindigkeit.

Der Komparator 85 weist zwei Operationsverstärker 121 und 122 auf. Der Operationsverstärker 121 hat im Gegenkopplungskreis einen Widerstand 123. Am negativen Eingang des Operationsverstärkers 121 ist ausserdem ein Widerstand 124 angeschlossen. An diesen Widerstand 124, den Signalleitungseingang des Komparators 85, ist der Ausgang des Weggebers 49 angeschlossen. Am negativen Eingang des Operationsverstärkers 121 ist ausserdem ein Widerstand 125 angeschlossen, der seinerseits an einen Spannungsteiler 126 angeschlossen ist. Der Ausgang des ersten Operationsverstärkers 121 ist an den negativen Eingang des zweiten Operationsverstärkers 122 angeschlossen, an dessen positivem Eingang, dem Steuerleitungseingang des Komparators 85, der Ausgang des Endstellungswertgebers 83, 84 angeschlossen ist. Durch diese Schaltung des ersten Operationsverstärkers 121 gibt er ein Summensignal aus dem Ausgangssignal des Weggebers 49 und dem Ausgangswert des Spannungsteiles 126 an den zweiten Operationsverstärker 122 ab. Der zweite Operationsverstärker gibt als der eigentliche Komparatorteil ein richtungsabhängiges Steuersignal ab. Über den Spannungsteiler 126 am negativen Eingang des ersten Operationsverstärkers 121 kann die Ausgangslage der Hubbewegung des Läufers 22 eingestellt werden.

Soweit vorstehend nichts anderes angegeben ist, ist davon auszugehen, dass der positive Eingang der abgehandelten Operationsverstärker mit der Erde verbunden ist.

**Patentansprüche**

1. Elektromagnetische Antriebsordnung mit Regelung (51, 52, 53, 54) einer Ausgangsgrösse einer durch einen eine Wicklung (31) aufweisenden Schwingankermotor (11) angetrieben, oszillierenden Verdrängerpumpe (10), bei der die Ausgangsgrösse erfasst, mit einem SOLL-Wert verglichen und der Strom durch die Wicklung (31) entsprechend der Abweichung gesteuert ist, dadurch gekennzeichnet, dass die Wicklung (31) eine auf dem Läufer (22) aufgebrachte Tauchspulenwicklung ist, die sich in einem vom Stator (21) gebildeten Magnetfeld bewegen kann, wobei an den von wenigstens einem Weggeber (49) abgetasteten Hubenden die Wicklung (31) umgepolt wird, und dass eine Ausregelung der Ausgangsgrösse während der Bewegung der Tauchspule innerhalb eines Hubes erfolgt.

2. Antriebsordnung nach Anspruch 1, dadurch gekennzeichnet, dass zwei Endstellungswertgeber (63, 64; 73, 74; 83, 84) für die beiden Umkehrstellungen des Läufers (22) und ein Richtungsumkehrschalter (66; 76; 86) vorgesehen sind (Fig. 4, 5, 6, 7).

3. Antriebsordnung nach Anspruch 2, dadurch gekennzeichnet, dass die Endstellungswertgeber (63, 64; 73, 74; 83, 84) an einem Komparator (65; 75; 85) angeschlossen sind, dessen dritter Eingang mit dem Weggeber (49) und dessen Ausgang mit dem Richtungsumkehrschalter (66; 76; 86) verbunden ist (Fig. 4, 5, 6, 7).

4. Antriebsanordnung nach Anspruch 3, dadurch gekennzeichnet, dass zwei Sollwertgeber (61, 62; 71, 72) für einen Hubparameter vorgesehen sind, die an den Richtungsumkehrschalter (66; 76) angeschlossen sind (Fig. 4, 5).

5. Antriebsanordnung nach Anspruch 3, dadurch gekennzeichnet, dass der Sollwertgerber (82) an beide Endstellungswertgeber (83, 84) und den Richtungsumkehrschalter (86) angeschlossen ist. (Fig. 6)

6. Antriebsanordnung nach einem der Ansprüche 1 bis 5, dadurch gekennzeichnet, dass der Weggeber (49) einen Schleifwiderstand (48) und eine Schleiffeder (47) aufweist.

7. Antriebsanordnung nach Anspruch 6, dadurch gekennzeichnet, dass zwischen dem Weggeber (49) und dem Vergleichsglied (77) eine Differenzierschaltung (70, 81) liegt.

8. Antriebsanordnung nach Anspruch 7, dadurch gekennzeichnet, dass zwischen dem Differenzierglied (70) und dem Vergleichsglied (77) ein zweites Differenzierglied (80) liegt.

9. Antriebsordnung nach einem der Ansprüche 1 bis 8, dadurch gekennzeichnet, dass der IST-Wertgeber ein Druckwandler (60) ist.

10. Antriebsanordnung nach einem der Ansprüche 1 bis 9, dadurch gekennzeichnet, dass der IST-Wertgeber ein Geschwindigkeitsgeber ist.

11. Antriebsanordnung nach Anspruch 10, dadurch gekennzeichnet, dass der Geschwindigkeitsgeber aus einer in einem Dauermagnetfeld bewegten Induktionsspule besteht.

12. Antriebsanordnung nach einem der Ansprüche 1 bis 11, dadurch gekennzeichnet, dass als Regler ein I-Regler verwendet ist.

13. Antriebsanordnung nach einem der Ansprüche 1 bis 11, dadurch gekennzeichnet, dass als Regler ein PI-Regler verwendet ist.

14. Antriebsanordnung nach einem der Ansprüche 1 bis 11, dadurch gekennzeichnet, dass als Regler ein PID-Regler verwendet ist.

15. Antriebsanordnung nach einem der Ansprüche 1 bis 14, bei der der Stator einen ringförmig geschlossenen Polschuh und einen mittig darin angeordneten Polkern aufweist, dadurch gekennzeichnet, dass der Läufer (22) am Polkern (26) geführt ist.

16. Antriebsanordnung nach Anspruch 15, dadurch gekennzeichnet, dass der Läufer (22) mittels zweier Gruppen von je drei Wälzlagern (36) am Polkern (26) geführt ist, von denen je eine Wälzlagergruppe an je einer der Stirnseiten des Läufers (22) angeordnet ist, wobei die Innenringe der Wälzlager (36) auf Lagerstiften (37a) sitzen, die mit dem Wicklungsträger (32) des Läufers (22) fest verbunden sind, und wobei die Aussenringe der Wälzlager (36) unmittelbar auf dem Polkern (26) laufen.

17. Antriebsanordnung nach Anspruch 15 oder 16, bei der die Pumpe eine zwischen zwei relativ zueinander bewegbaren Kragarmen des Antriebs eingespannte Balgpumpe ist, dadurch gekennzeichnet, dass der mit dem bewegbaren Teil (13) der Verdrängerpumpe (10) verbindbare Kragarm (35) mit dem Läufer (22) fest verbunden ist und sich von diesem aus rechtwinklig zu dessen Bewegungsbahn durch eine parallel zur Bewegungsbahn des Läufers (22) ausgerichtete Aussparung (27) im Stator (23) hindurch erstreckt.

18. Antriebsanordnung nach Anspruch 17, dadurch gekennzeichnet, dass zwei Kragarme (35) vorgesehen sind, je einer an einer der Stirnseiten (32) des Läufers (22), und dass ausserhalb des Stators (21) beide Kragarme (35) mit einem Bügel (39) verbunden sind, der seinerseits mit dem bewegbaren Teil (13) der Verdrängerpumpe (10) verbindbar ist.

19. Antriebsanordnung nach einem der Ansprüche 1 bis 18, dadurch gekennzeichnet, dass die Verbindung sowohl zwischen dem Stator-Kragarm (45) und dem feststehenden Teil (14) der Verdrängerpumpe (10) wie auch zwischen dem Läufer-Kragarm (42) und dem bewegbaren Teil (13) der Verdrängerpumpe (10) steckbar ausgebildet ist.

20. Antriebsanordnung nach einem der Ansprüche 1 bis 12, dadurch gekennzeichnet, dass die Verdrängerpumpe als Balgpumpe (10) mit Innendurchfluss des Fördermediums ausgebildet ist.

21. Antriebsanordnung nach Ansprüchen 19 und 20, dadurch gekennzeichnet, dass die Balgpumpe (10) an ihren beiden Enden (13, 14) einen Rücksprung (19, 20) und in beiden axialen Richtungen je einen daran anschliessenden Vorsprung (13, 17, 14, 18) aufweist.

22. Antriebsanordnung nach Anspruch 21, dadurch gekennzeichnet, dass der Rücksprung als umlaufende Rille (19, 20) und/oder die anschliessenden Vorsprünge als umlaufende Wülste (17, 18) ausgebildet sind.

23. Antriebsanordnung nach Anspruch 22, dadurch gekennzeichnet, dass die Enden (44, 46) der Kragarme (42, 45) gabelförmig sind.

## Claims

1. Electromagnetic driving mechanism including a control (51, 52, 53, 54) of an output variable of an oscillating displacement pump (10) driven by a motor (11) of the oscillating armature type including a coil (31), in which said output variable is detected and compared with a set value and the current through said coil (31) is controlled in accordance with the error signal, characterized in that said coil (31) is a plunger coil affixed to the runner (22) which is adapted to move in a magnetic field formed by the stator (21), changing polarity of the coil (31) at the stroke ends monitored by at least one displacement pick-up (49), and that a stabilising control of said output variable occurs during movement of said plunger coil within one stroke.

2. Driving mechanism according to claim 1, characterized in that two limit position pick-ups (63, 64; 73, 74; 83, 84) for the two reversing positions of the runner (22) and a direction reversing switch (66; 76; 86) are provided (figures 4, 5, 6, 7).

3. Driving mechanism according to claim 2, characterized in that said end position pick-ups (63, 64; 73, 74; 83, 84) are connected to a comparator (65; 75; 85) the third input of which is connected to said displacement pick-up (49) and the output of which is connected to said direction reversing switch (66; 76; 86) (figures 4, 5, 6, 7).

4. Driving mechanism according to claim 3, characterized in that two setvalue pick-ups (61, 62; 71, 72) for a stroke parameter are provided which are connected to said direction reversing switch (66, 76) (figures 4, 5).

5. Driving mechanism according to claim 3, characterized in that said set-value pick-up (82) is connected to both end position pick-ups (83, 84) and said direction reversing switch (86) (figure 6).

6. Driving mechanism according to any of the claims 1 to 5, characterized in that said displacement pick-up (49) includes a sliding resistor (48) and a sliding spring (47).

7. Driving mechanism according to claim 6, characterized in that a differentiating circuit (70, 81) is connected between said displacement pick-up (49) and said comparator (77).

8. Driving mechanism according to claim 7, characterized in that a second differentiating member (80) is connected between said differentiating member (70) and said comparator (77).

9. Driving mechanism according to any of the claims 1 to 8, characterized in that the pick-up for the actual value is a pressure transducer (60).

10. Driving mechanism according to any of the claims 1 to 9, characterized in that the pick-up for the actual value is a velocity sensor.

11. Driving mechanism according to claim 10, characterized in that said velocity sensor comprises a moving coil in a permanent magnetic field.

12. Driving mechanism according to any of the claims 1 to 11, characterized in that an integral type controller is used as the controller.

13. Driving mechanism according to any of the claims 1 to 11, characterized in that a proportional-integral type controller is used as the controller.

14. Driving mechanism according to any of the claims 1 to 11, characterized in that a proportional-integral-differential type controller is used as the controller.

15. Driving mechanism according to any of the claims 1 to 14, in which the stator comprises an annular, closed pole shoe and a pole core arranged centrally therein, characterized in that the runner (22) is guided at the pole core (26).

16. Driving mechanism according to claim 15, characterized in that said runner (22) is guided at said pole core (26) by means of two groups each comprising three anti-fricion bearings (36), one each of said anti-friction bearing groups being disposed on one each of the facing sides of the runner (22), the inner races of said anti-friction bearings (36) being located on bearing pins (37a) rigidly connected to the coil carrier (32) of the runner (22), the outer races of said anti-friction bearings (36) running directly on the pole core (26).

17. Driving mechanism according to claim 15 or 16, in which said pump is a bellow pump mounted between two cantilevered arms of the prime mover which are movable relative to each other, characterized in that the cantilever arm (35) adapted to be connected to the movable portion (13) of said displacement pump (10) is fixedly connected to said runner (22) and extends perpendicular to its path of movement through an opening (27) provided in the stator (23) and extending in parallel to the path of movement of the runner (22).

18. Driving mechanism according to claim 17 characterized in that there are provided two cantilevered arms (35) one each at one of the facing sides (32) of the runner (22), and that outside of the stator both cantilevered arms (35) are connected with a bail (39) which in its turn is adapted to be connected to the movable portion (13) of said displacement pump (10).

19. Driving mechanism according to any of the claims 1 to 18, characterized in that the connections both between the cantilevered arm (45) of the stator and the static portion (14) of said displacement pump (10), and between the cantilevered arm (42) of the runner and the movable portion (13) of said displacement pump (10) are of the plug-in type.

20. Driving mechanism according to any of the claims 1 to 12, characterized in that said displacement pump is designed as a bellows pump (10) with internal flow of the medium to be pumped.

21. Driving mechanism according to claim 19 and 20, characterized in that said bellows pump (10) includes at each of its two ends (13, 14) a reduced diameter portion (19, 20) between two larger diameter portions (13, 17, 14, 18) immediately following thereto in both axial directions.

22. Driving mechanism according to claim 21, characterized in that said reduced diameter portion is designed as an all around groove (19, 20) and/or the adjacent larger diameter portions are designed as all around bulges (17, 18).

23. Driving mechanism according to claim 22, characterized in that the ends (44, 46) of said cantilevered arms (42, 45) are of the fork type.

## Revendications

1. Dispositif de commande électromagnétique avec réglage (51, 52, 53, 54) d'une grandeur de sortie d'une pompe volumétrique oscillante (10) entraînée par un moteur (11) à armature oscillante et comportant un enroulement (31), dans lequel la grandeur de sortie est saisie, et est comparée à une valeur de consigne, le courant passant par l'enroulement (31) étant commandé en fonction de l'écart, caractérisé par le fait que l'enroulement (31) est constitué par l'enroulement d'une bobine mobile montée sur un équipage mobile (22) et capable de se déplacer dans le champ magnétique crée par le stator (21), la polarité de l'enroulement (31) étant inversée aux extrémités de la course détectée par au moins un capteur de déplacement, et qu'une régulation de la grandeur de sortie a lieu pendant le déplacement de la bobine mobile pendant une course.

2. Dispositif de commande selon la revendication 1, caractérisé par le fait qu'il est prévu deux générateurs (63, 64; 73, 74; 83, 84) de valeurs de positions d'extrémité pour les deux positions d'inversion de l'équipage mobile (22) et un commutateur d'inversion de direction (66; 76; 86) (figures 4, 5, 6, 7).

3. Dispositif de commande selon la revendication 2, caractérisé par le fait que les générateurs de valeurs de positions d'extrémité (63, 64; 73, 74; 83, 84) sont reliés à un comparateur dont la troisième entrée est reliée au capteur de déplacement (49) et dont la sortie est reliée aux commutateurs d'inversion de direction (66; 76; 86) (figures 4, 5, 6, 7).

4. Dispositif de commande selon la revendication 3, caractérisé par le fait qu'il est prévu deux générateurs de valeurs de consigne (61, 62; 71, 72) pour un paramètre de la course et reliés aux commutateurs d'inversion de direction (66; 76) (figures 4, 5).

5. Dispositif de commande selon la revendication 3, caractérisé par le fait que le générateur de valeurs de référence ou de consigne (82) est relié aux deux générateurs de valeur de position d'extrémité (83, 84) et au commutateur d'inversion de direction (86) (figure 6).

6. Dispositif de commande selon l'une des revendications 1 à 5, caractérisé par le fait que le capteur de déplacement (49) comporte une résistance à contact glissant (48) et un ressort glissant (47).

7. Dispositif de commande selon la revendication 6, caractérisé par le fait qu'entre le capteur de déplacement (49) et le dispositif comparateur (77) est prévu un circuit différenciateur (70, 81).

8. Dispositif de commande selon la revendication 7, caractérisé par le fait qu'entre le circuit différenciateur (70) et le dispositif comparateur (77) est prévu un second dispositif différenciateur (80).

9. Dispositif de commande selon l'une des revendications 1 à 8, caractérisé par le fait que le générateur de valeur instantané est un transducteur de pressions (60).

10. Dispositif de commande selon l'une des revendications 1 à 9, caractérisé par le fait que le générateur de la valeur instantanée est un générateur de signal de vitesse.

11. Dispositif de commande selon la revendication 10, caractérisé par le fait que le générateur de la valeur de vitesse est constitué par une bobine d'induction déplacé dans le champ d'un aimant permanent.

12. Dispositif de commande selon l'une des revendications 1 à 11, caractérisé par le fait que l'on utilise comme régulateur un régulateur intégral.

13. Dispositif de commande selon l'une des revendications 1 à 11, caractérisé par le fait que l'on utilise comme régulateur un régulateur proportionnel et intégral.

14. Dispositif de commande selon l'une des revendications 1 à 11, caractérisé par le fait que l'on utilise comme régulateur un système proportionnel, intégral et différentiel.

15. Dispositif de commande selon l'une des revendications 1 à 14, dans lequel le stator comporte une pièce polaire annulaire fermée et un noyau polaire disposé en son centre, caractérisé par le fait que l'équipage mobile (22) est guidé sur le noyau polaire.

16. Dispositif de commande selon la revendication 15, caractérisé par le fait que l'équipage mobile est guidé sur le noyau polaire à l'aide de groupes à trois paliers de roulement (36), parmi lesquels chaque groupe de roulement est disposé au niveau de l'une des faces frontales de l'équipage mobile (22), les bagues intérieures des roulements (36) sont montées sur des chevilles (37a) solidaires du support d'enroulement (32) de l'équipage mobile, et les bagues extérieures des roulements (36) roulant directement sur le noyau polaire.

17. Dispositif de commande selon la revendication 15 ou 16, dans lequel la pompe est une pompe à soufflet montée entre deux bras en consoles ou en porte-à-faux du dispositif, mobiles entre eux, caractérisé par le fait que le bras en console ou en porte-à-faux susceptible d'être relié avec la partie mobile (13) de la pompe volumétrique (10) est solidaire de l'équipage mobile (22) et s'étend perpendiculairement à la trajectoire de ce dernier, à travers une ouverture (27) ménagée dans le stator (23), parallèlement à la trajectoire de l'équipage mobile.

18. Dispositif de commande selon la revendication 17, caractérisé par le fait qu'il est prévu deux bras en console ou en porte-à-faux (35), un bras à chacun des côtés frontaux (32) de l'équipage mobile (22) et qu'à l'intérieur du stator (21), les deux bras en console ou en porte-à-faux (35) sont reliés à un étrier (39) qui, à son tour, est susceptible d'être relié à la partie mobile (13) de la pompe volumétrique (10).

19. Dispositif de commande selon l'une des revendications 1 à 18, caractérisé par le fait que la liaison entre le bras en console ou en porte-à-faux (45) du stator et la partie fixe (14) de la pompe volumétrique (10), de même qu'entre le bras-console ou en porte-à-faux (42) de l'équipage mobile et la partie mobile (13) de la pompe volumétrique (10) sont réalisées à la manière de liaisons à enfichage.

20. Dispositif de commande selon l'une des revendications 1 à 12, caractérisé par le fait que la pompe volumétrique est réalisée sous la forme d'une pompe à soufflet (10), à passage intérieur du fluide à transporter.

21. Dispositif de commande selon les revendications 19 et 20, caractérisé par le fait que la pompe à soufflet (10) comporte à ses deux extrémités (13, 14) une portion en retrait (19, 20) et dans chacune des deux directions axiales un élargissement saillant (13, 17, 14, 18) qui s'y raccorde.

22. Dispositif de commande selon la revendication 21, caractérisé par le fait que la partie en retrait est réalisée sous la forme d'une gorge (19, 20) et/ou que les élargissements saillants qui s'y raccordent sont réalisés sous la forme de bourrelets périphériques (17, 18).

23. Dispositif de commande selon la revendication 22, caractérisé par le fait que les extrémités (44, 46) des bras en console ou en porte-à-faux (42, 45), ont la forme d'une fourche.

# Fig.1

Fig. 2

Fig. 3

Fig. 4

0 016 023

Fig. 5

Fig. 6

17

Fig. 7